Europäisches Patentamt

European Patent Office

Office européen des brevets

(11) Publication number: 0 230 775
A1

## (12) EUROPEAN PATENT APPLICATION

(21) Application number: 86310066.5

(22) Date of filing: 23.12.86

(51) Int. Cl.⁴: **A41D 13/06** , A41D 13/08

(30) Priority: 31.12.85 GB 8531929

(43) Date of publication of application:
05.08.87 Bulletin 87/32

(84) Designated Contracting States:
AT BE CH DE ES FR GB GR IT LI LU NL SE

(71) Applicant: **Glennie, Kenneth Donald**
**17A Arduthie Road**
**Stonehaven, Kincardine, Deeside(GB)**

(72) Inventor: **Glennie, Kenneth Donald**
**17A Arduthie Road**
**Stonehaven, Kincardine, Deeside(GB)**

(74) Representative: **Pattullo, Norman et al**
**Ian G. Murgitroyd and Company Mitchell**
**House 333 Bath Street**
**Glasgow G2 4ER Scotland(GB)**

(54) **Protective device.**

(57) A protective device for fitment around a limb to prevent water contact with a dressing or the like on the limb, the device having a waterproof generally tubular body for enveloping the limb and a sealing portion secured to the body an an open end of the body for engaging and forming a waterproof seal with the limb to prevent water penetrating the inside of the body. Pre-fixing means in the form of an adhesive tab is provided for assisting in one-handed operation of the device by closing the open end of the body around the limb prior to implementing the sealing portion.

FIG.4

Xerox Copy Centre

## Protective Device

This invention relates to a protective device.

More specifically, this invention relates to a method for keeping dry, during bathing or showering, such body parts, primarily the limbs, that may be covered in dressings, plaster of Paris, adhesives or skin disorders. The device of the invention is also suitable for preventing the spread of infection in food preparation and handling units or similar industries. It would also be of benefit should there be some reason why the body part should be kept dry or protected from contact with materials or the local atmosphere.

In hospital and similar institutions, at present, patients are offered plastic bags which are then secured at one or both ends with elastic bands or similar constricting devices and showered in preference to a bath where the efficiency of such a device is questionable. This can bring hardships to patients especially the elderly with long standing chronic conditions. A common example would be varicose ulcers, and foot problems.

According to the present invention there is provided a protective device for fitment around a limb, comprising a first waterproof portion capable of extending around a limb and forming with the limb a waterproof seal, and a generally tubular second waterproof portion for enveloping an area of a limb, the first and second portions being conjoined in a waterproof manner.

The invention may be in the form of a readily expandable, premoulded covering in latex or similar material which will not only accommodate a dressing on a limb, but will also provide a watertight seal with the limb to prevent access of water to the dressing. The covering may be in various sizes and shapes depending on the size and shape of the patient's limb, and the use for which the device is intended. The waterproof seal may be effected by the tightening of the first waterproof portion around the limb to provide a seal similar to that provided by a surgeon's glove and may be enhanced by the use of a water repellant gel or jelly (for example, KY jelly).

Not all patients may be anatomically ideal for the basic device and in some cases a further constricting strap or the like may be required to assist the function.

The first portion may be of any of a range of materials such for example as latex or natural rubber, or synthetic rubber such as styrene-butadiene rubber, butadiene-acrylonitrile rubber, ethylene-propylene rubber, polychloroprene, polyisobutylene and polyurethane rubber. Other alternatives are cis-l,4-polybutadiene, poly(dimethylsiloxane) polysulphide rubber, polypropylene, polyethylene and polyvinyl chloride. These materials may if desired be coated or otherwise treated with anti-allergenic material.

The first portion is preferably of a width greater than I centimetre so that the area of waterproof seal with the limb provides a pathway for leakage of water too great to allow failure of the seal. The first portion may be in the form of a strip of latex or other material which is welded, heat-sealed or adhered to the second portion at one end of the second portion; such a strip may be wound around the limb so that each winding partially overlaps a previous winding, thereby producing an overall sealed area of substantial length along the limb.

The first portion is preferably resilient so that it can be tensioned around the limb.

The seal provided by the first portion may be achieved in several different ways; when in the form of a strip the first portion may be passed around the limb and secured to itself by, for example, adhesive, "Velcro" hook-and-eye fastening, buckle, stud, hook or clasp. A subsidiary pre-fixing device may also be provided to give initial closure of an open end of the second portion around the limb so that it becomes easier to fasten the main strip to effect the seal. The pre-fixing device is preferably a tab which is secured at one part of the second portion and capable of being fixed to another part of that portion. An area of the second portion in the vicinity of the pre-fixing device may preferably be of greater stiffness than the main part of the second portion, whereby controlled distortion of said area can be achieved on actuation of the pre-fixing device. The enhanced stiffness can be a function of the material of the second portion in that area or may be provided by an additional layer or coating on the second portion.

The second portion may be closed at one end so as to extend in the form of a bag over the limb. Alternatively the second portion may be open at both ends, a first portion being provided at each end to seal the device against the limb, thereby to leave one end of the limb free.

The second portion may be made from a wide range of materials, and may be of the same material as the first portion. Other possibilities are polyester, polyethylene, laminates, single-sided or double-sided metallised plastics, and polypropylene. The most preferred material is metallised polyethylene.

The second portion may be in the form of a sleeve of uniform diameter which is sufficiently wide to extend around most limbs, or may be shaped for particular dressings and limbs, for example by providing glove-like fingers for use of the device on hands and arms.

Embodiments of the present invention will now be described, by way of example, with reference to the accompanying drawings, in which:-

Fig. I is a side view of a protective device of the present invention, for use on a patient's leg;

Fig. 2 is a side view of a device of the invention for use in a patient's arm, shown in use;

Fig. 3 is a perspective view of an upper portion of the device of Fig. 2 showing its manner of manufacture;

Fig. 4 is a side view of a further device of the invention for use on any limb of a patient;

Fig. 5 is a front view of a patient's lower body showing the device of Fig. 4 in use on one leg and a further device of the invention in use on the patient's other foot; and

Fig. 6 is a front perspective view of an upper part of the second portion of a device of the present invention.

Referring to Fig. I of the drawings, the protective device of that embodiment of the invention is formed of latex rubber as used in a surgeon's glove.

The device illustrated in Fig. I is for use in covering the lower leg and has a first portion I which is highly elasticated such as to effect a watertight seal with the limb being covered. The watertight seal may be supplemented by the use of gel or jelly. In extreme cases, a further constricting device may be employed such as an elastic strap.

The portion 2 of the device is adapted to conform to the configuration of bulky dressings, plaster casts, bandages or in some cases an infected area of the skin.

The lowermost portion 3 in the embodiment illustrated is adapted to conform to the shape of a patient's foot.

The lowermost portion 3 may in certain applications be substituted for a second portion similar to the portion I such as to give a tubular device sealed at both ends.

It is envisaged that the device may be supplied in a sterile pack together with water repellant gel or jelly to enhance the performance of the device.

The device has as its prime function to maintain dressings dry during bathing but may also be used for hygienic purposes to protect infected areas or to prevent contact of the covered limb with foot straps etc.

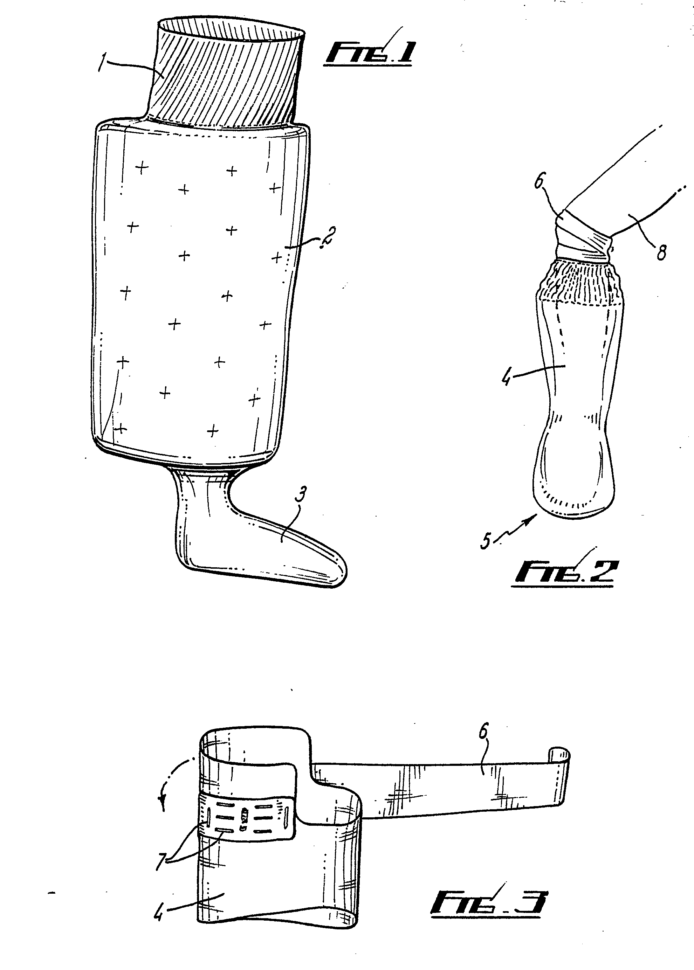

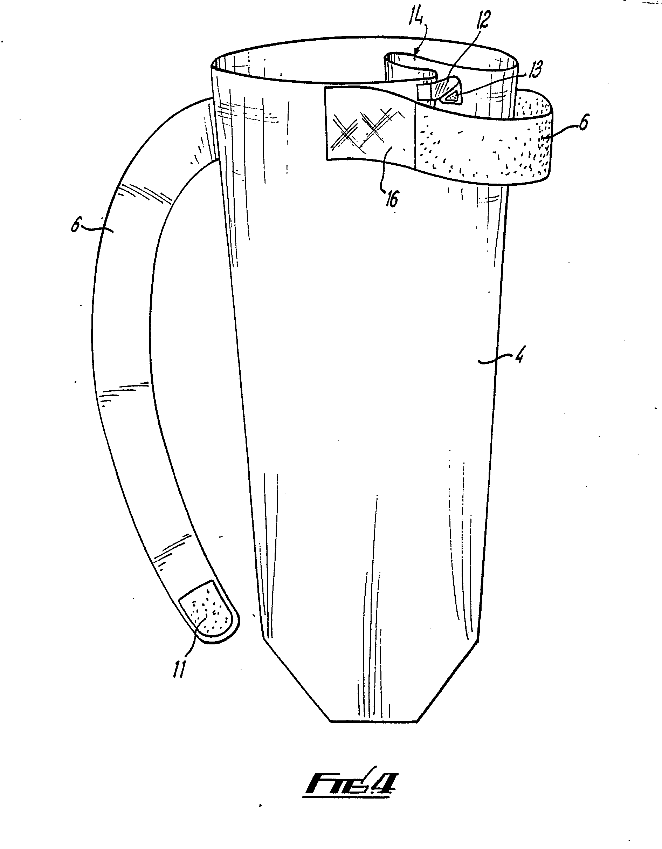

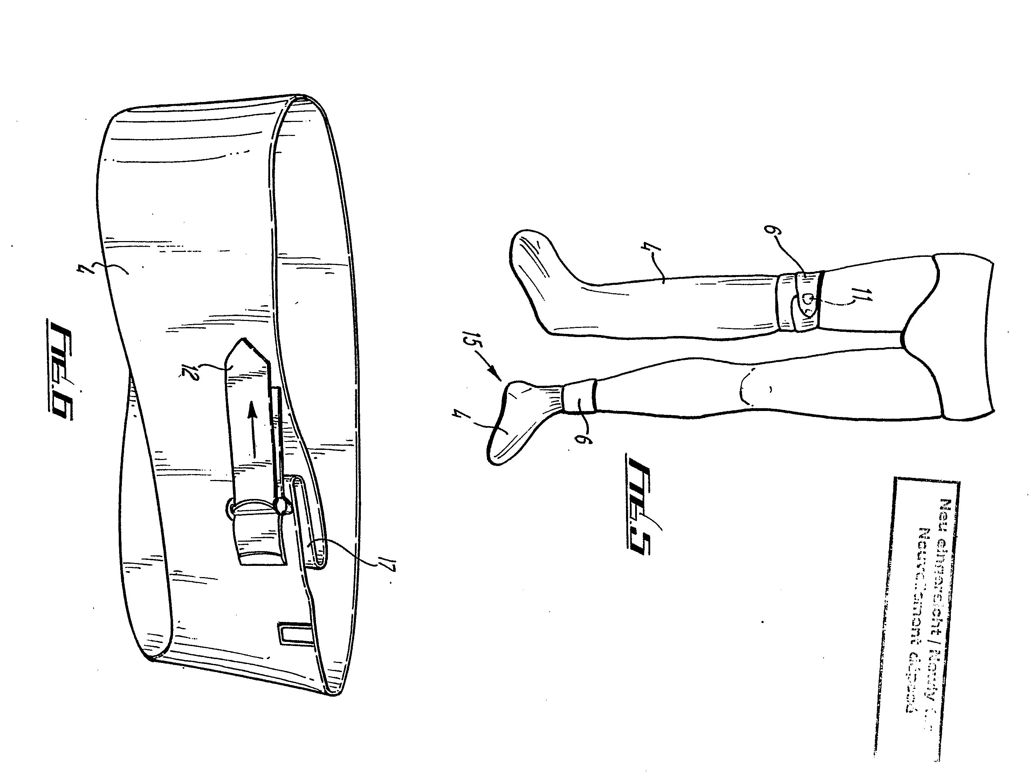

Referring now to Fig. 2, the device has two discrete portions, namely a sleeve portion 4 of metallised polyester closed at its lower end 5 and forming a bag to receive the forearm and hand of a patient, and a sealing portion 6 of latex which is attached to the sleeve portion 4 at its open upper end. The latex 6 is in the form of a strap one end of which is secured by heat sealing to the sleeve 4 in the manner shown in Fig. 3; the latex strap passes around the sleeve 4 whose upper portion is then folded over the strap 6 to envelop it. The strap 6 has holes 7 through it, and the polyester of the sleeve 4 seals to itself, either by heat or by adhesive, through the holes 7, thereby holding the strap 6 firmly in position.

The strap 6 in use is passed around the patient's arm 8 above the sleeve 4 under moderate tension and is then secured to itself by means of an adhesive pad (not shown) on the free end of the strap 6. The strap 6 then provides a waterproof seal with the patient's arm 8 as shown in Fig. 2.

In Figs. 4 and 5 a device of the invention for use on a patient's leg is shown, although the device of Fig. 4 may also be used on a patient's arm as the sleeve portion 4 is not specially shaped for any particular limb. The sleeve 4 in this case is made of metallised polyethylene and the latex strap 6, which is coated on both faces with a hypoallergenic low-tack adhesive, is secured to it through an intermediate member I6 of gauze-backed SELLOTAPE adhesive material. The latex of the strap 6 is incompatible with the metallised polyethylene of the sleeve 4 in that they cannot easily and effectively be adhered directly to one another, but both materials have been found to adhere satisfactorily to the material of the intermediate member I6. The gauze backing of the intermediate member I6 prevents the member from stretching sugnificantly under tension, and therefore avoids differential stretching of the member I6 and latex strap 6 which would cause failure of the adhesive connection between them.

The free end of the strap 6 has a pad II of double-sided adhesive tape. A short pre-fixing tension strap or tab I2 of metallised polyethylene is also provided at the upper part of the sleeve 4, being secured at one end by adhesive. The tension tab I2 also has at its free end a pad I3 of double-sided adhesive tape.

In use, the device of Fig. 4 receives a patient's limb (for example the leg as shown in Fig. 5) and the tension tab I2 is pulled around the sleeve 4 to provide a fold I4. The tab I2 is then adhered to the sleeve 4 by means of the pad I3 to maintain the fold and provide initial closure and tension in the upper part of the sleeve 4 around the patient's leg. The latex strap 6 is then wrapped helically under tension around the leg, with about a 50% overlap on previous windings, and the free end of the strap 6 is adhered to the previous winding of the strap 6 by the adhesive pad II. The device then provides a waterproof covering and seal around the patient's lower leg, with the latex strap 6 extending helically along the leg to provide a substantial length of seal. An important feature of the device in this embodiment of the invention is the provision of a stiffening member I7 in the vicinity of the tension tab I2. This considerably assists in the operation of the tab I2 by ensuring that the deformation of the upper part of the sleeve which occurs on actuation of the tab I2 takes place in a controlled manner, and with the alignment of the upper edge of the sleeve being reasonably maintained. It is important to provide such a control facility when the device may be applied to a limb using only one hand, as this greatly eases the operation.

In the present case the stiffening member I7 is in the form of a strip of adhesive plastics material applied to the sleeve 4.

Markings (not shown) may be provided on the latex strap 6 in order to guide a user of the device in applying the correct amount of tension to the strap 6. Such markings may be printed onto the strap 6 in the form of geometric designs whose proportions distort when the strap 6 is stretched. For example, elongate diamonds, as $\Diamond$ , or ovals, as $0$ , distort to produce squares, as $\diamondsuit$ , or circles, as $\circ$ , and the achievement of the latter indicates that the correct tension is being applied to the strap 6. In general terms, an elongation of the strap 6 to a degree of I9-27% is acceptable and does not result in a tourniquet effect on the limb.

In a modification of the device of Fig. 4 the open end of the sleeve can be provided with a buckle through which the latex strap 6 is passed on winding; the buckle provides an additional security for the strap.

The other device I5 shown in Fig. 5 is of similar construction to that of Fig. 4 but the sleeve portion 4 is shorter so as to be suitable for use on the foot of the patient.

Fig. 6 shows the operation of an alternative tension tab I2, in which a D-ring is provided on the sleeve 4 and the tab I2 is maintained under tension by passing through the D-ring instead of relying on the adhesive pad I3 as in Fig. 4. The area of the sleeve 4 adjacent the tab I2 is stiffened to assist in its folding.

In practice, the protective device of these embodiments of the invention have the latex strip wound round a mandrel, or cop, for easy winding of the strip around the limb, and also for neat and compact storage of the strip prior to use. It is of advantage for the cop to be generally cylindrical with a bulbous central portion for maintaining the greatest tension in the centre of the strip. This form of the cop is most useful when the latex strip has a coating of low-tack adhesive, and it maintains the strip flat on unwinding.

Modifications and improvements may be made without departing from the scope of the invention.

## Claims

1. A protective device for fitment around a limb, comprising a first waterproof portion capable of extending around a limb and forming with the limb a waterproof seal, and a generally tubular second waterproof portion for enveloping an area of a limb, the first and second portions being conjoined in a waterproof manner.

2. A protective device according to Claim I, wherein the first portion is in the form of an elongate strip one end of which is secured to the second portion.

3. A protective device according to Claim 2, wherein the first portion is resiliently expansible.

4. A protective device according to Claim 2 or 3, wherein the first portion has at its free end means for securing the free end to another part of the first portion.

5. A protective device according to any one of the preceding Claims, wherein the first and second protions are conjoined by mutual adhesion to an intermediate member.

6. A protective device according to any one of the preceding Claims, wherein the first portion is coated with a low-adhesion hypoallergenic material.

7. A protective device according to any one of the preceding Claims, wherein pre-fixing means is provided adjacent an open end of the second portion to provide a temporary closure of said open end around the limb.

8. A protective device according to Claim 7, wherein the pre-fixing means is a tab secured to one part of the second portion and having means for securing to another part of the second portion.

9. A protective device according to Claim 7 or 8, wherein an area of the second portion in the vicinity of the pre-fixing means is of greater stiffness than the main body of the second portion.

10. A protective device according to any one of the preceding Claims, wherein the second portion is open at one end only.

*FIG.1*

1

2

3

*FIG.2*

6

8

4

5

*FIG.3*

6

7

4

FIG 4

Neu eingereicht / Newly filed
Nouvellement déposé

**FIG.5**

**FIG.6**

## DOCUMENTS CONSIDERED TO BE RELEVANT

EP 86310066.5

| Category | Citation of document with indication, where appropriate, of relevant passages | Relevant to claim | CLASSIFICATION OF THE APPLICATION (Int. Cl.4) |
|---|---|---|---|
| A | US - A - 3 852 826 (SCHINDLER) <br> * Fig. 3 * <br> -- | 1-5, 7,8, 10 | A 41 D 13/06 <br> A 41 D 13/08 |
| A | US - A - 4 344 189 (FUTERE et al.) <br> * Fig. 1,14 * <br> -- | 1-4, 7,8 | |
| A | GB - A - 2 132 072 (K.R.ASSOCIATES INC.) <br> * Fig. * <br> -- | 1,3 | |
| A | DE - A1 - 3 301 854 (SCHWEIGER OTTO) <br> * Fig.; claims * <br> -- | 1-5, 7,10 | |
| A | DE - C - 851 783 (KARL OTTEN-SCHLAGER) <br> * Totality * <br> ---- | 1-5, 7,8, 10 | **TECHNICAL FIELDS SEARCHED (Int Cl.4)** <br><br> A 41 D |

The present search report has been drawn up for all claims

| Place of search | Date of completion of the search | Examiner |
|---|---|---|
| VIENNA | 12-03-1987 | NETZER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82